# EUROPÄISCHE PATENTSCHRIFT

(11) **EP 2 421 502 B1**
(45) Veröffentlichungstag und Bekanntmachung des Hinweises auf die Patenterteilung: **18.03.2015**
(21) Anmeldenummer: 10700803.9
(22) Anmeldetag: 08.01.2010
(51) Int. Cl.: A61K 8/63, A61Q 17/04, A61K 31/19, A61K 31/58, A61Q 19/00, A61Q 19/08

(54) **ZUSAMMENSETZUNG ENTHALTEND GLYCYRRHETINSÄURE ZUR REPARATUR BEREITS EINGETRETENER SCHÄDEN AN DER HAUTEIGENEN DNA**
COMPOSITION COMPRISING GLYCYRRHETIC ACID FOR REPAIRING PREVIOUSLY INCURRED DAMAGED TO SKIN DNA
COMPOSITION COMPRENANT l'ACIDE GLYCYRRHÉTINIQUE POUR RÉPARER DES DOMMAGES DÉJÀ SUBIS PAR L'ADN DE LA PEAU

(30) Priorität: 23.04.2009 DE 102009018334
(43) Veröffentlichungstag der Anmeldung: 29.02.2012
(73) Patentinhaber: Beiersdorf AG, 20253 Hamburg (DE)
(72) Erfinder: KOLBE, Ludger, 21255 Dohren (DE); SCHERNER, Cathrin, 22844 Norderstedt (DE); MANN, Tobias, 22175 Hamburg (DE); AHLHEIT, Sabrina, 22303 Hamburg (DE); HONG, Michael, 10789 Berlin (DE)
(86) Internationale Anmeldenummer: PCT/EP2010/000052
(87) Internationale Veröffentlichungsnummer: WO 2010/121676

(56) Entgegenhaltungen:
- EP-A1- 1 834 630
- EP-A1- 1 834 631
- DE-A1-102006 009 850
- DE-A1-102007 041 473
- FR-A1- 2 533 129

## Beschreibung

Die vorliegende Erfindung betrifft die Verwendung von Glycyrrhetinsäure zur Herstellung kosmetischer oder dermatologischer Zubereitungen zur Reparatur bereits eingetretener Schäden an der haüteigenen DNA.

Die Haut ist einer Vielzahl von Umwelteinflüssen ausgesetzt, die zu ihrer Schädigung führen können. Neben Lipiden und Proteinen ist dabei vor allem die DNA betroffen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Insbesondere Hautzellen erhalten durch häufige UV-Exposition einen hohen Anteil an DNA-Schäden und benötigen deshalb besonders effiziente und leistungsfähige Reparatursysteme. Nur effiziente hauteigene DNA-Reparatursysteme sind in der Lage, Schäden schnell zu beseitigen, wodurch Hautveränderungen und ein frühzeitiges Auftreten von Hautalterungserscheinungen verhindert werden können. Effiziente DNA-Reparatur trägt damit entscheidend zum Erhalt einer gesunden, vitalen Haut bei. Stimulierung und Unterstützung hauteigener Reparatursysteme durch kosmetisch-dermatologische Inhaltsstoffe sind deshalb sehr wichtig.

Die angesichts der großen Anzahl Nucleotide, die pro Zellteilung kopiert werden müssen, notwendige hohe Zuverlässigkeit bei der DNA-Replikation wird einerseits durch die Spezifität der DNA-Polymerasen, die an der Replikation beteiligt sind (Replicasen), andererseits durch deren zusätzlich vorhandene Exonucleaseaktivität garantiert: Sie katalysieren das Herausschneiden unpassender Nucleotide und deren Ersatz durch die richtigen sofort nach der Neusynthese. Den durch chemische Einwirkung entstehenden Schäden (durch Mutagene, von denen einige sich zwischen zwei übereinanderliegende Basenpaare der Doppelhelix einschieben können) sowie den von Strahlungseinwirkung herrührenden Veränderungen (wobei z. B. zwei übereinanderliegende Thymin-Reste dimerisieren können) helfen Reparatur-Enzyme (darunter: Endonucleasen, Polymerasen, Ligasen) ab, die unpassende Nucleotide wieder aus den DNA herausschneiden und ersetzen. Einige dieser Reparatursysteme sind induzierbar, d. h. sie werden erst bei Bedarf synthetisiert. Die Frage, welcher Strang die Original-Information enthält und welcher falsch synthetisiert wurde, wird vom Reparaturenzym offenbar anhand des Methylierungszustands entschieden.

Durch Exposition zu Umwelteinflüssen (z.B. UV-Strahlung, chemische und physikalische Faktoren) entstehen Schäden an den DNA-Molekülen der Zellen des Körpers, die jedoch durch zelleigene DNA-Reparaturmechanismen behoben werden können. Dabei erfolgt zuerst die Erkennung des.DNA-Schadens und dann die Reparatur des vorliegenden Schadens.

Werden die Schäden nicht vollständig kurz nach ihrer Entstehung durch Reparaturprozesse beseitigt, reichern sich diese in Form von permanenten DNA-Schäden in den Zellen an und werden nachfolgend an die Tochterzellen weitergegeben. Diese permanenten Schäden besitzen insbesondere durch mögliche Spätfolgen (schrittweise Funktionsausfälle) ein hohes Schadenspotential und spielen eine immer größere Rolle in Alterungsprozessen und bei der Schadensauslösung bereits im Kindesalter.

Aufgabe der vorliegenden Erfindung war es mithin, den Übelständen des Standes der Technik Abhilfe zu schaffen. Insbesondere sollten Wirkstoffe und Zubereitungen zur Verfügung gestellt werden, die zur Reparatur bereits eingetretener Schäden an der hauteigenen DNA dienen sollten.

Erstaunlicherweise werden diese Aufgaben gelöst durch die Verwendung von Glycyrrhetinsäure zur Herstellung kosmetischer oder dermatologischer Zubereitungen zur Reparatur bereits eingetretener Schäden an der hauteigenen DNA.

Erfindungsgemäß weisen Glycyrrhetinsäure ein hohes Potential zur Reparatur geschädigter DNA auf.

Die Verwindung von Glycyrrhetinsäure und Glycyrrhizin in Kosmetika und Dermatika ist an sich bekannt. Glycyrrhetinsäure ist durch folgende Struktur gekennzeichnet:

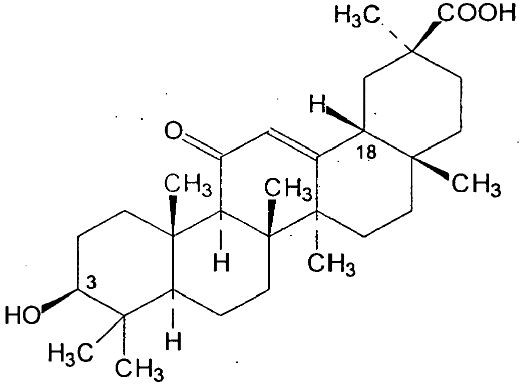

Neben der natürlich vorkommenden 18 β-Form existiert auch eine 18 α-Form. Die aus Süßholz (Glycyrrhiza glabra, Leguminosae) isolierbare Glycyrrhetinsäure ist das Aglycon von Glycyrrhizin (welches das 2 β-Glucuronido-α-glucuronid der Glycyrrhetinsäure darstellt).

Glycyrrhizin ist durch folgende Struktur gekennzeichnet:

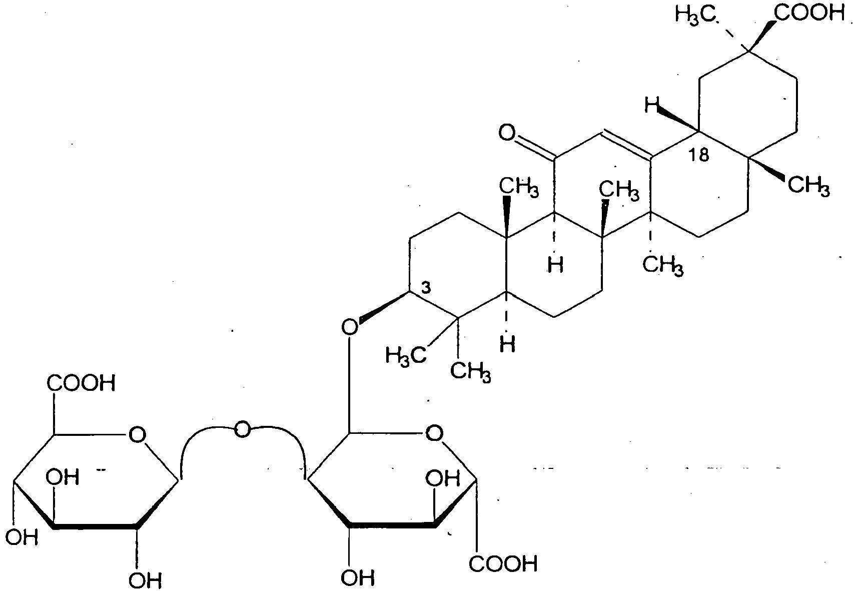

Glycyrrhetinsäure wird als entzündungshemmendes Mittel verwendet. Das Glycyrrhe-tinsäure-3-Hemisuccinat dient als Ulcustherapeutikum.

Glycyrrhizin wirkt entzündungshemmend und dient in Form von Süßholzsaft (Sirupus liquiritiae) als Hustenmittel und Expektorans..

Glycyrrhetinsäure und Glycyrrhizin sind dem Fachmann als Wirkstoffe mit einer hautaufhellenden Wirksamkeit bekannt. Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an erfindungsgemäß verwendeter Glycyrrhetinsäure.

Es kann aber auch erfindungsgemäß von Vorteil sein, ohne sol-che anderen Substanzen auszukommen, namentlich Flavonoide.

Es ist erfindungsgemäß vorteilhaft, den erfindungsgemäß verwendete Glycyrrhetinsäure bzw. kosmetischen oder dermatologischen Zubereitungen, Glycyrrhetinsäure enthaltend, Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die weiteren Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindungsgemäß vorteilhaft, Glycyrrhetinsäure und/oder deren Derivate in verkapselter Form darzureichen, z.B. in Kollagenmatrices und anderen üblichen Verkapseluogsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, Glycyrrhetinsäure in wäßrige Systeme bzw. Tensidzubereitungen zur Reinigung der Haut einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), EDTA, EGTA und deren Derivate, ungesättigte Fettsäuren und deren Derivate (z.B. γ-Linolensäure, Linolsäure, Ölsäure), Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat), Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe

Die Menge der vorgenannten Antioxidantien (eine oder mehrere,Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Myristylmyristate, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaürat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole einer Kettenlänge von 3 bis 30 C-Atomen, wie z.B. Octyldocecanol, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl; Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird. Beispielsweise können vorteilhaft gewählt werden Glycerylmonostearat, CetearylAlkohol, PEG-40 Rizinusöll + Natriumcetylstearylsulfat.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoesäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Ethylhexyl-2-cyano-3,3-diphenylacrylat (Octocrylen);
- Sulfonsäure-Derivate des 3-Benzylidencamphers, wie z.B. 4-(2-Oxo-3-bornylidenmethyl)benzolsulfonsäure, 2-Methyl-5-(2-oko-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die erfindungsgemäß in Kombination mit Glycyrrhetinsäure verwendet werden können, soll selbstverständlich nicht limitierend sein.

Gegenstand der Erfindung ist auch die Verwendung einer Kombination aus Glycyrrhetinsäure mit mindestens einem UVB-Filter bzw. die Verwendung einer Kombination aus Glycyrrhetinsäure mit mindestens einem UVB-Filter in einer kosmetischen oder dermatologischen Zubereitung.

Es kann auch von Vorteil sein, die erfindungsgemäß verwendeten Glycyrrhetinsäure mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 4-(tert.-Butyl)-4'-methoxydibenzoylmethan, 1-(4'-tert.Butylphenyl)-3-(4'-methoxyphenyl)propan-1,3-dion, um 1-Phenyl-3-(4'-isopropyl-phenyl)propan-1,3-dion sowie um 2,4-Bis-{[4-(2-ethyl-hexyloxy)-2-hydroxy]-phenyl}-6-(4-methoxyphenyl)-1,3,5-triazin.. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination von Glycyrrhetinsäure mit mindestens einem UVA-Filter als Antioxidans bzw. die Verwendung einer Kombination aus Glycyrrhetinsäure mit mindestens einem UVA-Filter als Antioxidans in einer kosmetischen oder dermatologischen Zubereitung.

Gegenstand der Erfindung ist auch die Verwendung einer Kombination aus Glycyrrhetinsäure mit mindestens einem UVA-Filter und mindestens einem UVB-Filter als Antioxidans bzw. die Verwendung einer Kombination aus Glycyrrhetinsäure mit mindestens einem UVA-Filter und mindestens einem UVB-Filter als Antioxidans in einer kosmetischen oder dermatologischen Zubereitung.

Kosmetische und dermatologischen Zubereitungen mit einem wirksamen Gehalt an Glycyrrhetinsäure können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der-Carbonate und Hydrogencarbonate.

Kosmetische Zubereitungen, die ein Hautreinigungsmittel darstellen, enthalten vorzugsweise mindestens eine anionische, nicht-ionische oder amphotere oberflächenaktive Substanz, oder auch Gemische aus solchen Substanzen, die Glycyrrhetinsäure im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Diese kosmetischen oder dermatologischen Zubereitungen können auch Aerosole mit den üblicherweise dafür verwendeten Hilfsmitteln darstellen.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate

Kosmetische Zubereitungen, die kosmetische Reinigungszubereitungen für die Haut darstellen, können in flüssiger oder fester Form vorliegen. Sie enthalten neben Glycyrrhetinsäure vorzugsweise mindestens eine anionische, nichtionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 04 Gew.-% in den Reinigungszubereitungen vorliegen, bezogen auf das Gesamtgewicht der Zubereitungen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die vorliegende Erfindung umfaßt auch ein kosmetisches Verfahren zum Schutze der Haut vor oxidativen bzw. photooxidativen Prozessen, das dadurch gekennzeichnet ist, daß man ein kosmetisches Mittel, welches eine wirksame Konzentration an Glycyrrhetinsäure enthält, in ausreichender Menge auf die Haut aufbringt.

Ebenso umfaßt die vorliegende Erfindung auch ein Verfahren zum Schutze kosmetischer oder dermatologischer Zubereitungen gegen Oxidation oder Photooxidation, wobei diese Zubereitungen z.B. Schminkprodukte wie z.B. Lippenstifte, Teintgrundlagen, Wasch- und Duschzubereitungen, Cremes zur Behandlung oder Pflege der Haut oder um sämtliche anderen kosmetischen Zubereitungen darstellen, deren Bestandteile Stabilitätsprobleme aufgrund von Oxidation bzw. Photooxidation bei der Lagerung mit sich bringen können, dadurch gekennzeichnet, daß die kosmetischen Zubereitungen einen wirksamen Gehalt an Glycyrrhetinsäure aufweisen.

Vorzugsweise beträgt die Menge an Glycyrrhetinsäure in diesen Zubereitungen 0,01 - 10 Gew.-%, bevorzugt 0,05 - 5 Gew.-%, insbesondere 0,1 - 2,0 Gew.-%, bezogen auf das Gesamtgewicht der Zubereitungen.

Gegenstand der Erfindung ist auch das Verfahren zur Herstellung der erfindungsgemäßen kosmetischen Mittel, das dadurch gekennzeichnet ist, daß man in an sich bekannter Weise Glycyrrhetinsäure in kosmetische und dermatologische Formulierungen einarbeitet.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### O/W-Beispielrezepturen 1-5:

| **INCI - Bezeichnung** | **Gew.-%** | **Gew.-%** | **Gew.-%** | **Gew.-%** | **Gew.-%** |
|---|---|---|---|---|---|
| Glycerylstearatcitrat | | 2,00 | | | |
| Polyglyceryl-3 Methylglucosedistearat | | | | 2,25 | |
| Sorbitanstearat | | | | 0,75 | |
| Stearinsäure | 2,50 | | | | |
| Glyceryl Stearate SE | | | 1,00 | | |
| PEG-40 Stearat | | | | | 1,00 |
| Behenylalcohol | | 1,20 | | | |
| C12-15 Alkylbenzoat | 4,65 | 2,50 | 7,50 | 2,50 | 3,00 |
| Caprylsäure/Caprinsäuretriglyceride | 2,50 | 2,50 | | 2,50 | 2,50 |
| Carbomer | 0,10 | 0,10 | | 0,10 | 0,10 |
| Cetearyl Alkolhol * PEG-40 Rizinus Öl + Natrium Cetearyl Sulfat | | | 1,50 | | |
| Cetylstearylalcohol | | | | | 3,00 |
| Cetylalcohol | 2,00 | 2,00 | 2,50 | | |
| Cyclomethicon | | 2,15 | | 2,15 | 2,15 |
| Dicaprylylcarbonat | 2,00 | 2,50 | | 2,50 | 2,50 |
| Dimethicone | 0,35 | 0,35 | | 0,35 | 0,35 |
| Glycerin | 5,00 | 5,00 | 1,50 | 7,50 | 7,50 |
| Glycerylstearat | 1,20 | | | | 3,00 |
| Methylparaben | 0,20 | 0,20 | 0,20 | 0,20 | 0,20 |
| MyristylMyristate | | | 2,00 | | |
| Octyldocecanol | | | 4,00 | | |
| Phenoxyethanol | 0,40 | 0,40 | 0,50 | 0,40 | 0,40 |
| Propylparaben | 0,10 | 0,10 | | 0,10 | 0,10 |
| Stearyl Alkohol | 2,00 | | | 1,00 | |
| Trinatrium-EDTA | | | | 1,00 | 1,00 |
| Tapiocastärke | 1,50 | 1,00 | | 2,50 | |
| Talkum | | | | | 2,00 |
| Glycyrrhetinsäure | 0,15 | 0,10 | 0,10 | 0,10 | 0,10 |
| NaOH | q.s. | q.s. | | q.s. | q.s. |
| Parfüm | q.s. | q.s. | q.s. | q.s. | q.s. |
| 2-Ethylhexyl-2-cyano-3,3-diphenyl-acrylat | 1,50 | | 5,00 | | |
| Ethylhexylmethoxycinnamat | | | | 5,00 | 5,00 |
| Butylmethoxydibenzoylmethan | | | 1,50 | 2,00 | 2,00 |
| Phenylbenzimidazol Sulfonsäure | 1,00 | | 0,50 | | 3,00 |
| 2,4-Bis-{[4-(2-ethyl-hexyloxy)-2-hydroxy]-phenyl}-6-(4-methoxyphenyl)-1,3,5-triazin | | | 2,00 | | 1,00 |
| Xanthan Gummi | | | 0,10 | | |
| Wasser | ad 100 | ad 100 | ad 100 | ad 100 | ad 100 |

### O/W-Beispielrezepturen 6-11:

| **INCI - Bezeichnung** | **Gew.-%** | **Gew.-%** | **Gew.-%** | **Gew.-%** | **Gew.-%** | **Gew.-%** |
|---|---|---|---|---|---|---|
| Acrylat/C 10-30 Alkyl Acrylat Crosspolymer | | | | | 0,25 | |
| Ammonium Acryloyldimethyltaurat/VP Copolymer | 0,20 | 0,10 | | 0,25 | 0,10 | 0,20 |
| Butylmethoxydibenzoylmethan | | | | 2,00 | 2,00 | 1,00 |
| C12-15 Alkylbenzoat | 2,25 | 2,50 | 2,50 | 3,00 | | 2,50 |
| Caprylsäure/Caprinsäureglyceride | | 1,50 | | | | |
| Caprylsäure/Caprinsäure-triglyceride | 2,50 | 2,50 | 2,50 | 2,00 | | 2,25 |
| Carbomer | | 0,20 | 0,2 | | | |
| Cetearyl Alkohol | 3,00 | 3,00 | 3,00 | | | 1,00 |
| Cetyl Alkohol | | | | | | 1,00 |
| Cyclomethicone | 2,15 | 2,15 | 2,15 | 1,00 | 10,00 | 2,50 |
| Dimethicone | 0,35 | 0,35 | 0,35 | 1,00 | 3,00 | 0,50 |
| Ethylhexylmethoxycinnamat | | | | 5,00 | 5,00 | 3,00 |
| Glycerin | 5,00 | 5,00 | 5,00 | 5,00 | 7,50 | 5,00 |
| Glycerylisostearat | 3,00 | | | | | |
| Glycerylstearat | | | | 2,00 | | |
| Glycerylstearat SE | | | | | | 1,00 |
| Glycyrrhetinsäure | 0,10 | 0,10 | 0,10 | 0,10 | 0,10 | 0,10 |
| Methylparaben | 0,20 | 0,20 | 0,20 | 0,15 | 0,10 | 0,10 |
| Octyldodecanol | 2,50 | 2,50 | 2,50 | 3,00 | | 1,00 |
| PEG-40 Stearat | | 1,00 | 1,00 | | | |
| Parfüm | q.s. | q.s. | q.s. | q.s. | q.s. | q.s. |
| Phenoxyethanol | 0,40 | 0,40 | 0,40 | 0,25 | 0,20 | 0,40 |
| Polyglyceryl-3-Methylglucose Distearat | 2,00 | | | | | |
| Polysorbat 40 | | | 0,50 | | | |
| Propylparaben | 0,10 | 0,10 | 0,10 | 0,05 | 0,10 | 0,10 |
| Natriumcetylstearylsulfat | | | | 1,50 | | 1,25 |
| NaOH | q.s. | q.s. | q.s. | q.s. | q.s. | q.s. |
| Natriumstearoylglutamat | | | | | 0,20 | |
| Natriumpolyacrylat | 0,10 | | | | | |
| Sucrose Polystearat + Hydriertes Polyisobuten | | | | | 1,00 | |
| Talkum | 1,00 | 1,00 | | | | |
| Tapiokastärke | | 1,00 | 2,00 | | | |
| Wasser | ad 100 | ad 100 | ad 100 | ad 100 | ad 100 | ad 100 |
| Xanthan Gummi | 0,10 | 0,10 | 0,10 | 0,20 | | 0,10 |

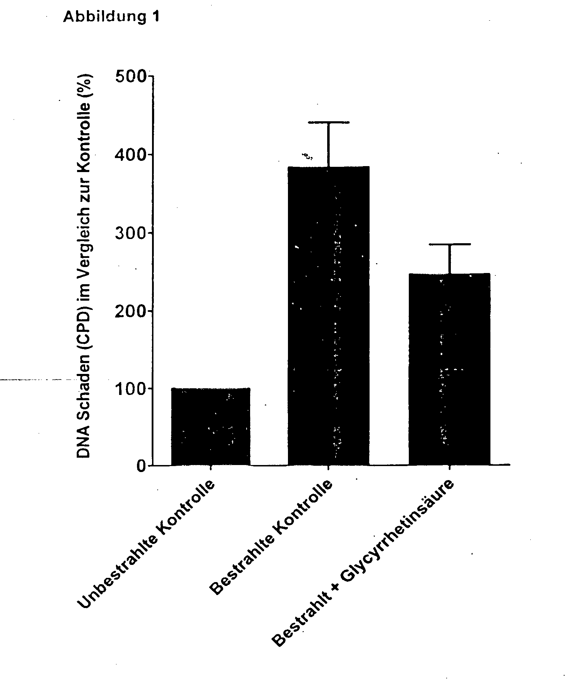

### Abb. 1 Verbesserte Reparatur von DNA-Schäden durch Glycyrrhetinsäure in vivo:

### Siehe Abbildung 1

In einer Anwendungsstudie wurde 16 Probanden 2-mal täglich eine erfindungsgemäße Öl-in-Wasser-Zubereitung mit 0,1% Glycyrrhetinsäure, 57% Wasser und 13% Öl auf die Prüfareale appliziert. Nach 2 Wochen wurden die Areale mit 0,75 MED sonnen-simuliertem Licht bestrahlt und anschließend Saugblasen gezogen. Die Zellkerne der Epidermiszellen der Saugblasendächer wurden mittels eines floureszenz-gekoppelten Anti-Cyclo-Pyrimidin-Dimer (CPD)-Antikörpers markiert und die Floureszenz der Zellkerne im Cytometer bestimmt.

Die Behandlung mit Glycyrrhetinsäure führt zu einer beschleunigten Reparatur des UVinduzierten DNA-Schadens.

## Patentansprüche

1. Dermatologische Zubereitungen zur Reparatur von durch UV-Strahlung hervorgerufenen Schäden an der hauteigenen DNA enthaltend einen wirksamen Gehalt an Glycyrrhetinsäure.

2. Dermatologische Zubereitung zur Reparatur von durch UV Strahlung hervorgerufenen Schäden an der hauteigenen DNA gemäß Anspruch 1 **dadurch gekennzeichnet, daß** die Glycyrrhetinsäure in topischen dermatologischen Zubereitungen in Konzentrationen von 0,01 - 10 Gew.-%, bevorzugt 0,05 - 5 Gew.-%, insbesondere 0,1 - 2,0 Gew.% bezogen auf das Gesamtgewicht der Zubereitungen, vorliegt.

## Claims

1. Dermatological preparations for repairing damage to skin DNA caused by UV radiation, comprising an effective content of glycyrrhetic acid.

2. Dermatological preparation for repairing damage to skin DNA caused by UV radiation according to Claim 1, **characterized in that** the glycyrrhetic acid is present in topical dermatological preparations in concentrations of 0.01-10% by weight, preferably 0.05-5% by weight, in particular 0.1-2.0% by weight, based on the total weight of the preparations.

## Revendications

1. Compositions dermatologiques pour la réparation de dégâts provoqués sur l'ADN propre à la peau par un rayonnement UV, contenant une teneur efficace en acide glycyrrhétinique.

2. Composition dermatologique pour la réparation de dégâts provoqués sur l'ADN propre à la peau par un rayonnement UV selon la revendication 1, **caractérisée en ce que** l'acide glycyrrhétinique se trouve dans des compositions dermatologiques topiques en des concentrations de 0,01 - 10% en poids, de préférence de 0,05 - 5% en poids, en particulier de 0,1 - 2,0% en poids, par rapport au poids total des compositions.
